Europäisches Patentamt

European Patent Office

Office européen des brevets

(11) Publication number: **0 409 156 A1**

## EUROPEAN PATENT APPLICATION

(21) Application number: 90113645.7

(22) Date of filing: 17.07.90

(51) Int. Cl.⁵: **C12N 15/04, C12P 21/02,** C12N 15/81

The applicant has filed a statement in accordance with Rule 28 (4) EPC (issue of a sample only to an expert). Accession number(s) of the deposit(s): FERM BP-2404, FERM BP-2406 and FERM BP-2405.

(30) Priority: 19.07.89 JP 188173/89

(43) Date of publication of application:
**23.01.91 Bulletin 91/04**

(84) Designated Contracting States:
**BE CH DE DK ES FR GB IT LI NL SE**

(71) Applicant: **THE GREEN CROSS CORPORATION**
3-3, Imabashi 1-chome Chuo-ku
Osaka-shi Osaka 541(JP)

(72) Inventor: **Okabayashi, Ken, c/o The Green Cross Corporation**
Central Research Lab., 2-1180-1, Shodaiohtani
Hirakara-shi, Osaka 573(JP)
Inventor: **Ohi, Hideyuki, c/o The Green Cross Corporation**
Central Research Lab., 2-1180-1, Shodaiohtani
Hirakara-shi, Osaka 573(JP)

Inventor: **Hirabayashi, Kazumoto, c/o The Green Cross Corp.**
Central Research Lab., 2-1180-1, Shodaiohtani
Hirakara-shi, Osaka 573(JP)
Inventor: **Miura, Masami, c/o The Green Cross Corporation**
Central Research Lab., 2-1180-1, Shodaiohtani
Hirakara-shi, Osaka 573(JP)
Inventor: **Shimizu, Miho, c/o The Green Cross Corporation**
Central Research Lab., 2-1180-1, Shodaiohtani
Hirakara-shi, Osaka 573(JP)
Inventor: **Kawabe, Haruhide, c/o The Green Cross Corporation**
Central Research Lab., 2-1180-1, Shodaiohtani
Hirakara-shi, Osaka 573(JP)

(74) Representative: **von Kreisler, Alek, Dipl.-Chem. et al**
**Patentanwälte Von Kreisler-Selting-Werner, Deichmannhaus am Hauptbahnhof**
**D-5000 Köln 1(DE)**

(54) **Fused yeast for foreign protein expression, methods of producing said yeast and methods of producing foreign proteins.**

(57) The fused yeasts possessing the capabilities of producing foreign proteins of both parent yeasts, comprising a first yeast capable of producing a foreign protein wherein a DNA sequence encoding the foreign protein has been incorporated in the chromosome thereof, and a second yeast capable of producing a foreign protein wherein a DNA sequence encoding the foreign protein has been incorporated in the chromosome thereof, their production methods and methods of producing foreign proteins are disclosed. According to the present invention, noticeable improvement of foreign protein production can be expected.

# FUSED YEAST FOR FOREIGN PROTEIN EXPRESSION, METHODS OF PRODUCING SAID YEAST AND METHODS OF PRODUCING FOREIGN PROTEINS

## BACKGROUND OF THE INVENTION

The present invention relates to a yeast capable of mass-producing a single foreign protein or two or more different foreign proteins, a method of producing said yeast and a method of producing a foreign protein using said yeast.

Development of recombinant DNA technology has made it possible to produce a wide variety of useful polypeptides using microorganisms. A large number of mammalian polypeptides, for example, human growth hormone and interferon, have already been produced using various microorganisms. This technology has made it possible to produce various useful polypeptides using microorganisms. Specifically, it has become possible to produce various proteins such as vaccines, hormones, enzymes and antibodies using microorganisms.

However, standard protein preparations derived from microorganisms, particularly from Escherichia coli , often contain contaminant endotoxin, which should be removed from the desired protein products.

It is a well-known fact that mammalian cells are difficult to be proliferated on such a large scale that protein secreted by cells can be produced at a low cost with high efficiency. The generation time of mammalian cells is considerably longer than that of microorganisms. Therefore, long-term cultivation is necessary to reach a sufficiently high cell concentration. Also, the cell concentration obtained by mammalian cell cultivation is considerably lower than that generally reached in large scale production of microorganisms. Moreover cell line improvement is more difficult than in microorganisms.

Since all eucaryotes have a mechanism of genetic information expression, it is assumed that any eucaryotic gene would be expressed more efficiently in eucaryotic hosts than in procaryotes such as Escherichia coli . The easiest-to-handle of the eucaryotes suitable for use is yeast.

It is easy to cultivate yeast since it is a microorganism. The amount of cells obtained per unit volume of culture broth is considerably higher in the yeast than in Escherichia coli . In addition, the fermentative behavior of the yeast is well understood, and the conditions for large scale fermentation thereof have already been firmly established. Furthermore, yeast cells contain no endotoxin.

For these reasons, it is evident that it is advantageous to produce a foreign protein with the yeast by means of well developed techniques of industrial microbiology and recently developed recombinant DNA technology.

In the meantime, sporal crossing is a known method of yeast breeding. Crosses have been made between sporulating Saccharomyces cerevisiae and strains related thereto.

In recent years, protoplast fusion has drawn attention as a method of yeast breeding not based on crossing. Accordingly, attempts have been made to breed non-sporulating yeasts and to breed interspecific hybrid yeasts.

It was in 1977 when the first report was presented of a study on cell fusion using yeast protoplasts. Since then, research into intra- and interspecific fusions in various yeast species has been extensive. Cell fusion can occur irrespective of sexual and species differences. Also, not only intercellular (interprotoplastic) fusion but also fusion with organelle such as mitochondria is possible.

## SUMMARY OF THE INVENTION

It is an object of the present invention to provide a novel fused yeast obtained by fusing yeasts transformed by gene recombination technology.

It is another object of the present invention to provide a method of producing said novel fused yeast.

It is still another object of the present invention to provide a method of producing a single or two or more species of foreign proteins with high efficiency.

Heretofore, no attempts have been known to fuse yeasts transformed by gene recombination technology that produce a useful protein.

Particularly, there is no report of an attempt to fuse yeasts transformed with the aim of producing a useful protein wherein the foreign gene that encodes the useful protein has been inserted into the chromosome of the host yeast.

It is also unknown to use the fused yeast thus obtained to increase the amount of foreign protein expressed or to obtain a number of foreign proteins from the same cell.

## BRIEF DESCRIPTION OF THE DRAWING

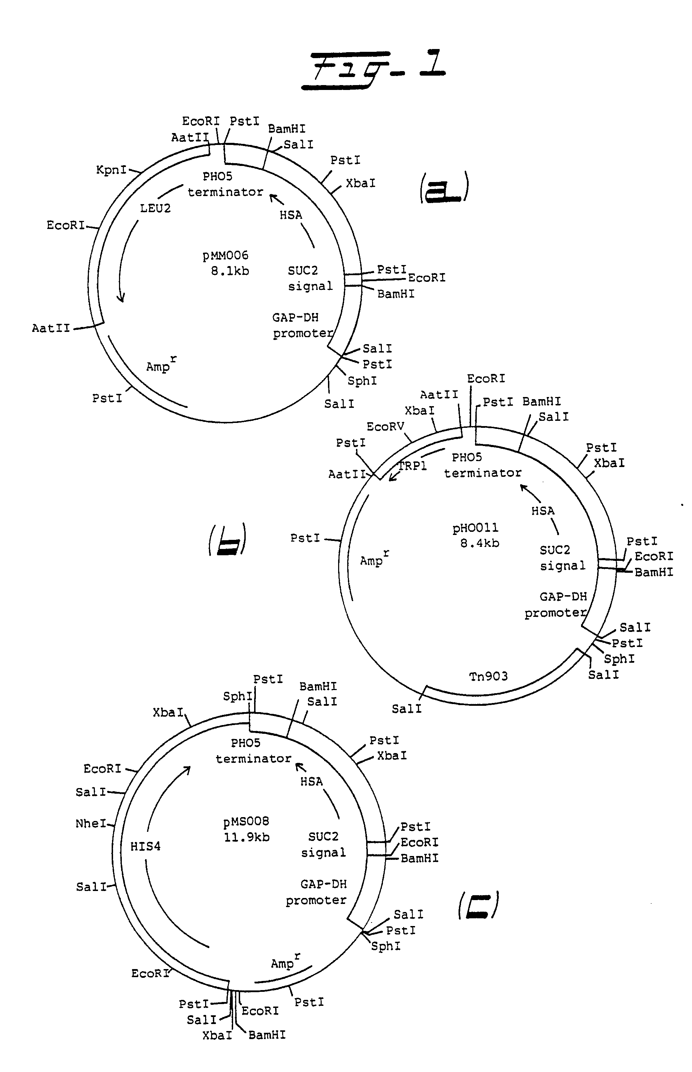

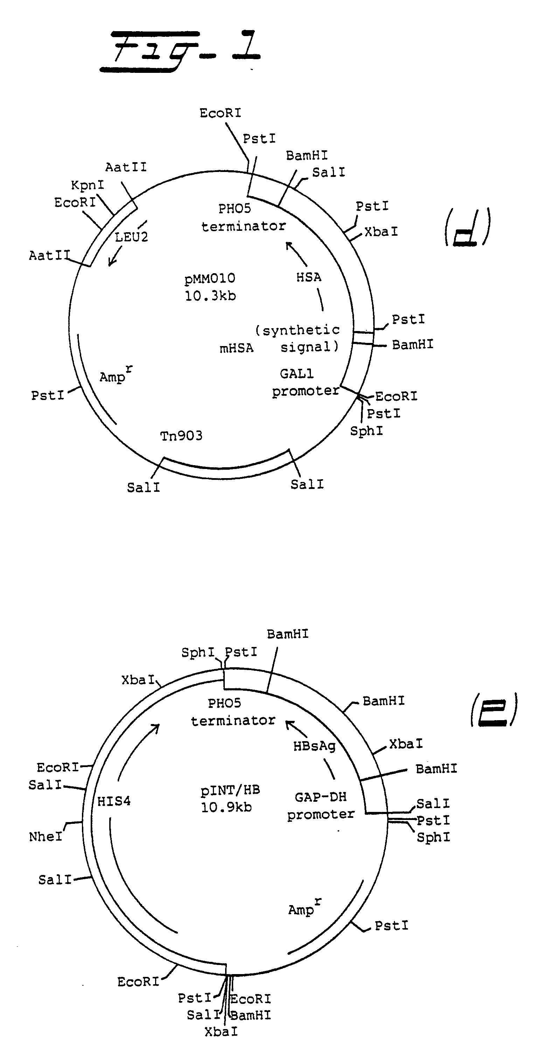

Fig. 1 (a) through (e) show plasmids pMM-006, pHO-011, pMS-008, pMM-010 and pINT/HB, respectively.

## DETAILED DESCRIPTION OF THE INVENTION

Accordingly, the present invention essentially comprises the following aspects:

(1) A fused yeast possessing the capabilities of producing a foreign protein of both parent yeasts, comprising a first yeast capable of producing a foreign protein wherein a DNA sequence encoding the foreign protein has been incorporated in the chromosome thereof, and a second yeast capable of producing a foreign protein wherein a DNA sequence encoding the foreign protein has been incorporated in the chromosome thereof.

(2) A method of producing a fused yeast capable of producing a foreign protein of both parent yeasts, characterized by fusion between a first yeast capable of producing a foreign protein wherein a DNA sequence encoding the foreign protein has been incorporated in the chromosome thereof and a second yeast capable of producing a foreign protein wherein a DNA sequence encoding the foreign protein has been incorpo rated in the chromosome thereof.

(3) A method of producing a foreign protein characterized in that the fused yeast described in (1) above is cultivated to produce a foreign protein, which is then harvested.

The present invention is hereinafter described specifically.

The plasmid used to produce a yeast for the present invention contains a partial DNA sequence of a gene present in the chromosome of the host yeast (e.g. LEU2, HIS4, TRP1, URA3 and ribosome DNA genes). The homologous sequence makes it possible to stably introduce the whole plasmid or a linear fragment thereof into the chromosome of the host yeast by recombination. In other words, the offspring cells can stably retain the introduced genetic material even in the absence of selection pressure during proliferation.

For example, a plasmid containing both a naturally occurring sequence of a chromosomal gene of a yeast and a foreign gene can be stably incorporated into the locus of the above-mentioned chromosomal gene.

Usable sequences homologous to the chromosome sequence of the host yeast include amino acid or nucleic acid synthetic pathway genes, ribosome DNA and Ty factor. Particularly, an amino acid or nucleic acid synthetic pathway gene can be used as a transformant selection marker since it serves as a gene to complement the variation in the host when the host yeast is an amino acid or nucleic acid auxotroph, that is, a strain lacking the amino acid or nucleic acid synthetic pathway gene. In this case, it provides a prototrophic property for the auxotrophy of the host yeast. Examples of the amino acid or nucleic acid synthetic pathway gene include LEU2, HIS4, TRP1 and URA3.

When the host yeast is an auxotroph as above, an amino acid or nucleic acid synthetic pathway gene can serve as a yeast selection gene marker. When the host yeast is an antibiotic-sensitive strain, a gene that expresses corresponding antibiotic resistance can serve for this purpose, and examples of such genes include those providing resistance to antibiotics such as cycloheximide, G418, chloramphenicol, bleomycin and hygromycin.

In the present invention, the foreign protein is not subject to limitation. Examples of preferable foreign proteins include human serum albumin (HSA), HBsAg, interferon-$\alpha$, -$\beta$ or -$\gamma$, urokinase, growth hormone, insulin and various lymphokines.

Genes of such foreign proteins are disclosed in, for example, Japanese Patent Publication Open to Public Inspection Nos. 29985/1987 (human serum albumin), 52883/1988 (HBsAg), 185189/1986 (interferon-$\alpha$), 108397/1986 (interferon-$\gamma$) and 180591/1985 (urokinase).

In these references, the genes are disclosed as plasmids containing a foreign protein gene.

Promoter is derived from the genomic DNA of a yeast, preferably Saccharomyces cerevisiae . It is preferable to use the promoter of a highly expressive yeast gene to express the foreign protein. Usable promoters include the promoter of TRPI gene, ADHI or ADHII gene, acidic phosphatase (PHO3 or PHO5) gene or isocytochrome C gene, the promoter of galactose metabolic pathway (GAL1, GAL10 or GAL7), the promoter of invertase (SUC2), or the promoter of a gene encoding a glycolytic pathway enzyme such as enolase, glyceraldehyde-3-phosphate dehydrogenase (GAPDH), 3-phosphoglycerate kinase (PGK), hexokinase, pyruvate decarboxylase, phosphofructokinase, glucose-6-phosphate isomerase, 3-phosphoglycerate mutase, pyruvate kinase, triose phosphate isomerase, phosphoglucose isomerase and glucokinase, or the promoter of the yeast conjugation pheromone gene encoding a-factor or α-factor.

Also, the plasmid cannot replicate itself in the host yeast, i.e. it contains substantially no region for autonomous replication initiation in the host yeast, such as the 2 μm DNA replication initiation region or ARS region (autonomous replicating sequence).

In a preferred embodiment, a signal sequence may be introduced into the plasmid. Usable signal sequences include yeast-derived signal sequences such as yeast invertase (Japanese Patent Publication Open to Public Inspection No. 41488/1985) and the α-factor gene (Japanese Patent Publication Open to Public Inspection No. 132892/1984). Also usable is a signal sequence specially synthesized for the purpose of secretionary expression in yeast (Japanese Patent Application No. 306674/1987 or 33657/1988).

Introduction of such a signal sequence causes the gene product to enter the secretionary pathway after foreign gene expression, and then it is transported to periplasmic space. Furthermore, it is possible to cause the gene product to be secreted into the medium via cell wall, which in turn permits considerable increase in yield. Moreover, since cytoclasis is unnecessary, recovery process simplification is possible.

Also, the plasmid contains an appropriate terminator for the termination of transcription, such as PHO5 or GAP-DH terminator.

This plasmid may contain a replication origin and genetic selection marker for bacterial host, particularlly Escherichia coli , in addition to the region encoding the foreign protein and the region homologous to the chromosome of the host yeast. Use of a replication origin and genetic selection marker for Escherichia coli in yeast hybrid vector offers some advantages. First, a large amount of hybrid vector DNA can be obtained by proliferation and replication in Escherichia coli . Second, hybrid vector construction can be easily achieved by using all cloning techniques based on Escherichia coli . Escherichia coli plasmids such as pBR322 contain an Escherichia coli replication origin and Escherichia coli genetic marker that provides resistance to antibiotics such as tetracycline and ampicillin, and can be used advantageously as part of yeast hybrid vector.

Accordingly, the present plasmid contains a promoter, a foreign protein encoding region controlled by the promoter, and a terminator for the termination of transcription that follows the region, and also contains a sequence homologous to the chromosome sequence of the host yeast. Also, the present plasmid may contain a signal sequence for secretionary production, a yeast selection gene marker, an Escherichia coli replication origin and an Escherichia coli selection gene marker as desired, and it contains substantially no yeast replication initiation region.

In the present invention, a yeast belonging to the genus Saccharomyces or Pichia is used as a host, with preference given to an auxotroph or antibiotic-sensitive strain.

The method of producing a transformant using this recombinant plasmid is as follows:

The recombinant plasmid is introduced into the chromosome of the host yeast cell. Specifically, it is desirable to cleave a sequence in the plasmid to be inserted, which is homologous to the chromosome of the host yeast cell, at an aribitrary site by restriction enzyme treatment and introduce the linearized plasmid into the host. The linearized plasmid is incorporated into the chromosome of the host yeast cell at the chromosome region homologous to the plasmid region. Linearized plasmids, in comparison with circular plasmids, are more frequently incorporated in the host chromosome. The host yeast used here is preferably a mutant involving a variation complemented by the yeast selection marker gene harbored in the plasmid to be inserted, e.g. Saccharomyces cerevisiae AH22 strain (a, his 4, leu 2, can 1), a leucine/histidine-requiring auxotrophic and G418-sensitive mutant.

Transformation of the host yeast cell is carried out by a known method such as the protoplast polyethylene glycol method or the electropolation method.

Next, it is deter mined whether or not the plasmid has been introduced into the target site, and whether the introduced gene is stable. Specifically, it is confirmed that the plasmid has been introduced into the target site by Southern blotting using as a probe a sequence homologous to the chromosome sequence of the host yeast cell used for transformation. It is also confirmed that the stability of the gene encoding the foreign protein remains unchanged even after the transformant is cultivated in nonselective medium over scores of generations by rating foreign protein production and recovery and retention of auxotrophy as indexes.

If these aspects are confirmed, the strain is identified as a transformant wherein the plasmid containing the desired foreign protein encoding region has been incorporated in the target site in the chromosome of the host yeast cell. This transformant, as a host, can be again transformed with a plasmid containing said desired protein encoding region. In this case, the region homologous to the chromosome of the host yeast cell may be a homologous region other than the region used for the first transformation.

Other examples of sequences homologous to the chromosome of the host yeast cell include ribosome DNA and Ty factor (transposon of yeast element). Since these genes exist in a plurality per cell, a number of desired genes can be incorporated into the host chromosome in a single transformation.

An example incorporation method is described below, but it is nothing more than a representation of a preferable means, and it is not to be construed as limitative. The homologous sequence site is replaceable by selective repetition.

The host should be Saccharomyces cerevisiae AH22 (mutant involving a variation both in the leucine synthetic pathway gene LEU2 and in the histidine synthetic pathway gene HIS4), a leucine/histidine-requiring auxotrophic and G418-sensitive mutant.

First, the host cell is transformed with a plasmid having LEU2, the gene for making the host free of leucine-requirement, in a sequence homologous to the chromosome sequence of the host yeast. The transformant thus obtained incorporates at the LEU2 gene locus on the chromosome a plasmid containing a foreign protein encoding region, and is a non-leucine-requiring strain, i.e. a strain capable of proliferating in leucine-free medium.

Next, this transformant, as a host, is transformed with a plasmid having HIS4, the gene for making the host free of leucine-requirement, in a sequence homologous to the chromosome sequence of the host yeast cell (of course also having a foreign protein encoding region). The transformant thus obtained incorporates at the HIS4 gene locus on the chromosome a plasmid containing a foreign protein encoding region, and is a non-histidine-requiring strain, i.e. a strain capable of proliferating in histidine-free medium. Now, the desired expression gene, namely, the foreign protein gene, has been introduced at two sites of LEU2 and HIS4.

Next, the non-leucine- and non-histidine-requiring transformant obtained above, as a host, is transformed with a plasmid having TRP1 in a sequence homologous to the chromosome sequence of the host yeast cell. This plasmid contains a G418 resistance gene as well as a foreign protein encoding region. The transformant thus obtained incorporates at the TRP1 gene locus on the chromosome a plasmid containing a foreign protein encoding region and a G418 resistance gene, and shows resistance to antibiotic G418. Thus, this transform ant contains a foreign protein gene at a total of three gene loci, namely, the LEU2, HIS4 and TPR1 gene loci on the chromosome. Here, the order of insertion does not count.

If a number of auxotrophs or strains that show resistance to a number of antibiotics can be obtained as hosts, a number of useful genes can be introduced into the regions correspon ding thereto.

As stated above, it is possible to insert the desired gene into a number of regions on the host chromosome. These genes incorporated in chromosome are not dislocated but stably retained, and in addition, incorporation of a multiplicity of genes enables mass production of the desired product.

Fusion of yeast transformants having on their chromosome a foreign protein encoding gene is carried out by a known method such as the protoplast fusion method [Pieter van Solingen et al.: J. Bacteriology, 130 , 946 (1977)]. Fused cells can be screened using as indexes recovery of auxotrophy and provision of antibiotic resistance.

The transformant is cultivated in a known medium. Examples of the medium include YPD liquid medium [1% yeast extract (Difco Laboratories), 2% Bactopeptone (Difco Laboratories), 2% glucose] and YPG liquid medium (the same composition as YPD liquid medium except that 2% glucose is replaced by 2% galactose).

The present invention is hereinaf ter described in more detail by means of the following working examples and reference examples, but the invention is not by any means limited by them.

Note that many of the techniques, reactions and analytical methods used for the present invention are obvious to those skilled in the art. Unless otherwise stated, all enzymes used for the present invention are commercially available, for example, from Takara Shuzo Co., Ltd., Japan; New England Biolabs (NEB), Massachusetts, USA; Amerscham, UK; and Bethesda Research Laboratories (BRL), Maryland, USA.

The buffers and reaction conditions for the enzyme reactions were used in accordance with the recommendations of the manufacturers of respective enzymes, unless otherwise stated.

Transformation of Escherichia coli using plasmids, plaque hybridization, electrophoresis and recovery of DNA from gel were carried out in accordance with the methods described in "Molecular Cloning", Cold Spring Harbor Laboratory (1982). Transformation of yeasts was carried out in accordance with the method described in "Method in Yeast Genetics", Cold Spring Harbor Laboratory (1981).

Fusion of yeasts was carried out in accordance with the method described in "the Journal of Bacteriology", 130 , 946 (1977).

HSA production and HBsAg production were measured as follows:

Measurement of HSA production

The supernatant obtained by 72-hr shaking culture in YPD or YPGal medium was assayed for HSA content by the ELISA or RPHA method.


(Outline of the ELISA method)

Antibody [anti-HSA F(ab')₂ fragment (goat), Code No. 0301-2221, produced by Cappel] was 4000 fold diluted with a 0.05 M sodium carbonate buffer (pH 9.5) (antibody concentration: about 2 μg/ml). This dilution was placed in an ELISA plate at 100 μl per well, and the antibody was adsorbed.
A 100 μl sample was placed in each well.
After addition of antibody (peroxidase-coupled IgG fraction, goat anti-HSA, produced by Cappel), a chromogenic liquid was added, and the resulting pigment was measured quantitatively at 492 nm.


Measurement of HBSAg production

After 72-hr shaking culture in YPD medium, centrifugation was conducted. Then, cells were collected, washed once with physiological saline, and suspended in a buffer [50 mM Tris-HCl (pH 7.5), 1 mM EDTA]. The resulting suspension was treated at a level of 10 using the ultrasound generator UR-200P, produced by Tomy Seiko, with ice cooling, for 9 minutes, after which it was centrifuged at 0°C and 13000 x g for 10 minutes. The supernatant thus obtained was assayed for HBsAg activity by the RPHA method using Antihebscell ® produced by The Green Cross Corporation, Japan.


Reference Example 1


[1] Cloning of GAPDH promoter region, GAL promoter region, SUC 2 signal region, LEU 2 region, HIS 4 region, TRP 1 region and PHO 5 terminator region, and preparation of HSA gene, HBsAg gene, synthetic signal region and G-418 resistance gene

These were each prepared by the methods described in the following references or methods analogous thereto, or ob tained commercially.
GAPDH promoter: Holland, H. J. and Holland, J. P., J. Biol. Chem., 254 , 12, 5466 (1979); Holland, H. J. and Holland, J. P., J. Biol. Chem., 254 , 19, 9839 (1979), Japanese Patent Publication Open to Public Inspection No. 84498/1988.
GAL 1 promoter and synthetic signal: Japanese Patent Application No. 103339/1988.
HBsAg gene: Japanese Publication Open to Public Inspection No. 52883/1988.
SUC 2 signal sequence: Japanese Patent Publication Open to Public Inspection Nos. 41488/1985 and 84498/1988.
HSA gene: Japanese Patent Publication Open to Public Inspection No. 29985/1987.
PHO 5 terminator: Japanese Patent Publication Open to Public Inspection No. 151183/1987.
G-418 resistance gene: Oka, A., Sugisaki, H. and Takanami, M., J. Mol. Biol., 147 , 217 (1981); Jimenez, A. and Davies, J., Nature, 287 , 869 (1980); Japanese Patent Publication Open to Public Inspection No. 40793/1986.
TRP 1: Derived from plasmid pBTI-10 (commercially available from Behringer Mannheim AG).
LEU2: Derived from plasmid pBTI-1 (commercially available from Behringer Mannheim AG).
HIS 4: Donahue, T. F., Daves, R. S. et al., Cell, 32 , 89 (1983).
Escherichia coli replication initiation region and ampicillin resistance gene: pUC 19 (obtained from Takara Shuzo Co., Ltd.) and pAT153.


[II] Construction of plasmids

Plasmids pMM-006, pMS-008, pHO-011, pMM-010 and pINT/HB were each constructed from pUC19 or

EP 0 409 156 A1

pAT153 in accordance with the method described in "Molecular Cloning", Cold Spring Harbor Laboratory (1982) (see Fig. 1 (a) through (e)).

Plasmid pMM-006 contains the leucine synthetic pathway gene LEU2 in a sequence homologous to the chromosome sequence of the host yeast cell, and an SUC 2 signal sequence, an HSA structural gene and a PHO 5 terminator are ligated under the control of GAP-DH promoter.

Plasmid pMS-008 contains the histidine synthetic pathway gene HIS4 in a sequence homologous to the chromosome sequence of the host yeast cell, and an SUC 2 signal sequence, an HSA structural gene and a PHO 5 terminator are ligated under the control of GAP-DH promoter.

Plasmid pHO-011 contains the tryptophan synthetic pathway gene TRP1 in a sequence homologous to the chromosome sequence of the host yeast cell, and an SUC 2 signal sequence, an HSA structural gene and a PHO 5 are ligated terminator under the control of GAP-DH promoter, and also contains a G418 resistance gene as a yeast selection marker gene.

Plasmid pMM-010 contains the leucine synthetic pathway gene LEU2 in a sequence homologous to the chromosome sequence of the host yeast cell, and a synthetic (mHSA) signal sequence, an HSA structural gene and a PHO 5 terminator are ligated under the control of GAL1 promoter, and also contains a G418 resistance gene.

Plasmid pINT/HB contains the leucine synthetic pathway gene LEU2 in a sequence homologous to the chromosome sequence of the host yeast cell, as ligated with an HBsAg structural gene and a PHO 5 terminator under the control of GAP-DH promoter.

Plasmid pMM-006, pMS-008 and PHO-011 have been internationally deposited at the Fermentation Research Institute of the Agency of Industrial Science and Technology of the Ministry of International Trade and Industry of Japan since April 28, 1989 as follows:

1) Name of microorganism: pMM006/ E. coli JM109 Accession number: FERM BP-2404
2) Name of microorganism: pMS008/ E. coli JM109 Accession number: FERM BP-2406
3) Name of microorganism: pH0011/ E. coli HB101 Accession number: FERM BP-2405

[III] Transformation of S. cerevisiae AH22 strain with plasmid pMM-006 (preparation of yeast transformant TMM-21)

Saccharomyces cerevisiae AH22 strain was used as a host.

Saccharomyces cerevisiae AH22 strain shows conjugation of the a-type, involving a variation in the histidine synthetic pathway gene (his 4) and the leucine synthetic pathway gene (leu 2). Thus, it cannot proliferate unless histidine and leucine are added to culture broth.

HSA secretionary expression plasmid pMM-006 was introduced into the chromosome of the yeast Saccharomyces cerevisiae AH22 strain as follows:

Saccharomyces cerevisiae AH22 was subjected to shaking culture in 50 ml of YPD medium (prepared by dissolving 10 g of yeast extract and 20 g of Bactopeptone in water, diluting this solution with water to reach a total quantity of 900 ml, autoclaving this dilution, and mixing it with separately autoclaved 100 ml of 20% glucose) at 37° C overnight, followed by centrifugation. Cells were collected and suspended in 20 ml of water, followed by recentrifugation Cells were collected and suspended in 10 ml of 50 mM dithiothreitol, 1.2 M sorbitol and 25 mM EDTA, pH 8.5, followed by gentle shaking at 30° C for 10 minutes. After centrifugation, cells were collected and suspended in 10 ml of 1.2 M sorbitol, followed by recentrifugation. Cells were collected and suspended in 10 ml of 1.2 M sorbitol, followed by centrifugation. Cells were collected and suspended in 10 ml of 0.2 mg/ml zymolyase 100T, 1.2 M sorbitol, 10 mM EDTA and 0.1 M sodium citrate, pH 5.8, followed by gentle shaking at 30° C for 1 hour. After centrifugation, cells were collected and washed with 10 ml of 1.2 M sorbitol and then with 10 ml of 10 mM calcium chloride and 10 ml of 1.2 M sorbitol, followed by centrifugation. Cells were collected and suspended in 1 ml of 10 mM calcium chloride and 1.2 M sorbitol. A 100 µl portion of this sus pension was transferred to a sterile test tube and mixed with 5 µl of a DNA solution containing 5 µg of pMM-006 linearized by digestion at the unique Kpηl site on the LEU 2 gene. This mixture was kept standing at room temperature for 15 minutes. Then, 1.2 ml of 20% polyethylene glycol 4000, 10 mM calcium chloride and 10 mM Tris-HCl, pH 7.5, were added. After gentle mixing, the resulting mixture was kept standing at room temperature for 20 minutes. After centrifugation, cells were collected and suspended in a YPD medium containing 0.1 ml of 1.2 M sorbitol and 10 mM calcium chloride, followed by gentle shaking at 30° C for 30 minutes. This suspension, in amounts of 1.5, 10, 20 and 50 µl, was mixed with 10 ml of agar medium incubated at 45° C and was spread over a plate of leucine-free medium. After solidification, the plate was kept standing and incubated at 30° C for 3 days. The resulting colony was taken by means of a tooth pick and suspended in 3 ml of 0.7%

7

yeast nitrogen base and 2% glucose, followed by shaking culture at 30°C for 2 days. After a 1.5 ml portion of this suspension was centrifuged, cells were collected and suspended in 3 ml of YPD medium (prepared by dissolving 10 g of yeast extract and 20 g of Bactopeptone in water, diluting this solution with water to reach a total quantity of 900 ml, autoclaving this dilution, and mixing it with 100 ml of separately autoclaved 20% dextrose), followed by shaking culture at 30°C. The HSA concentration of the resulting culture supernatant was measured by the RPHA method. A maximum HSA concentration of 20 $\mu$g/ml was detected at 3 day S following initiation of shaking culture.

The transformant thus obtained was named TMM-21.

[IV] Screening of transformant TMM-21 (LEU 2 gene stability and HSA production)

1) Location of the HSA gene by Southern blotting revealed that it had been introduced in the LEU 2 region in the chromosome.

2) The stability of the HSA gene was determined on the basis of HSA production and leucine-non-requiring property. Even after about 60 generations of cultivation in nonselective medium, the HSA gene was retained at a retention rate of 100%.

Reference Example 2

Yeast transformant TMS-34 was obtained in the same manner as in Reference Example 1 using plasmids pMS-008 and pHO-011 as constructed in Reference Example 1 and Saccharomyces cerevisiae AH22 strain as a host.

Reference Example 3

Yeast transformant THBS was obtained in the same manner as in Reference Example 1 using plasmid PINT/HB as constructed in Reference Example 1 and Saccharomyces cerevisiae AH22 strain as a host.

Example 1

The properties of the parent strains used for cell fusion in Example 1 are as shown in Table 1.

[I] Preparation of protoplasts of TMS-34 and TMM-21

TMS-34 was subjected to shaking culture in 50 ml of YPD medium (prepared by dissolving 10 g of yeast extract and 20 g of Bactopeptone in water, diluting this solution with water to reach a total quantity of 900 ml, autoclaving this dilution, and mixing it with 100 ml of separately autoclaved 20% glucose) at 37°C overnight. The resulting TMS-34 culture broth was centrifuged. TMS-34 cells were collected and suspended in 20 ml of water, followed by recentrifugation. TMS-34 cells were collected and suspended in 10 ml of 50 mM dithiothreitol, 1.2 M sorbitol and 25 mM EDTA, pH 8.5, followed by gentle shaking at 30°C for 10 minutes. After centrifugation, TMS-34 cells were collected and suspended in 10 ml of 1.2 M sorbitol, followed by recentrifugation. TMS-34 cells were collected and suspended in 10 ml of 1.2 M sorbitol, followed by centrifugation. TMS-34 cells were collected and suspended in 10 ml of 0.2 mg/ml zymolyase 100T, 1.2 M sorbitol, 10 mM EDTA, 0.1 M sodium citrate, pH 5.8, followed by gentle shaking at 30° for 1 hour. After centrifugation, TMS-34 cells were collected and washed with 10 ml of 1.2 M sorbitol and then with 10 ml of 10 mM calcium chloride and 10 ml of 1.2 M sorbitol. After centrifugation, TMS-34 protoplasts were collected.

TMM-21 protoplasts were prepared in the same manner as above.

[II] Cell fusion (fusion of TMS-34 and TMM-21)

Protoplastic TMS-34 and TMM-21 were each suspended in 0.5 ml of 1.2 M sorbitol and 10 mM CaCl₂. These suspensions of the two bacterial strains to be fused, each in an amount of 0.1 ml, were dispensed into a test tube, and kept standing at room temperature for 15 minutes. After addition of 2.4 ml of a polyethylene glycol solution [40%(w/v) polyethylene glycol 4000, 10 mM CaCl₂ and 10 mM Tris-HCl (pH 7.5)], the mixture was kept standing at room temperature for 20 more minutes.

After centrifugation, cells were collected in the form of a pellet, which was then suspended in 0.4 ml of an expression solution [1.2 M sorbitol, 10 mM CaCl₂ in YPD]. After shaking culture at 30°C for 30 minutes, the suspension was mixed with an agar solution and spread over minimal medium [yeast nitrogen base (W/O), produced by Difco Laboratories, glucose], followed by incubation at 30°C for several days to yield a fused yeast (referred to as FKO-1). The total colony count was 222.

The fused yeast was separated on a selection medium free of leucine and histidine. The single colony of the phenotype of LEU⁺, HIS⁺ was separated and suspended in sterile water. This colony was found to have G418 resistance as shown in Table 2 (fused cell markers). Thus, the strain obtained as this colony is identified as a fused yeast formed from the above-mentioned two yeast strains. The reason for this conclusion is as follows:

TMS-34, a parent strain of the fused yeast FKO-1, is an integrant derived from AH22 strain (a, leu2-3, leu2-112, his4-519, can1) wherein the HSA gene has been incorporated in the TRP1 and HIS 4 gene loci thereof. Since the G418 resistance gene was used as a gene marker for incorporation in the TRP1 gene locus, the phenotype of this strain is expected to be leu⁻, HIS⁺, G418ʳ. TMM-21, the other parent strain, is an integrant derived from AH22 strain wherein the HSA gene has been incorporated in the LEU2 gene locus thereof, and its phenotype is expected to be LEU⁺, his⁻, G418ˢ. Consequently, the phenotype of the strain occurring due to fusion of the above-mentioned combination of two parent strains is expected to be LEU⁺, HIS⁺, G418ʳ.

Immediately after being isolated as a single colony, the fused yeast of the present invention (FKO-1) was subjected to shaking culture at 30°C for 72 hours in a test tube containing YPD medium, followed by measurement of HSA production by the ELISA method. The results are summarized in Table 3. Table 3 also shows the results of measurement of HSA production by the ELISA method after shaking culture of the parent strains TMS-34 and TMS-21 under the same conditions as above. As is evident from these results, the fused cell showed an increased HSA production in comparison with its parent strains.

Also measured was HSA production of the fused yeast after 8 subcultures (once daily) on a plate of YPD medium, a nonselective medium (30°C for 72 hours). The results are shown in Table 4. As is evident from these results, HSA production showed almost no reduction even after long-term subculture in the nonselective medium.

Also, 10 single clones were isolated from the clones formed after 8 subcultures (once daily) on a plate of YPD medium, a nonselective medium, and marker stability was determined. The results are shown in Table 5. As is evident from these results, the marker gene was stably retained even after long-term subculture in the nonselective medium.

Example 2

The properties of the parent strains used for cell fusion in Example 2 are as shown in Table 1.

Protoplasts of TMS-34 and TMM-23 were fused to yield fused cells of TMS-34 and TMM-23 (referred to as FKO-3). The total colony count was 206.

The fused cell was separated on a selection medium free of leucine and histidine. The single colony of the phenotype of LEU⁺, HIS⁺ was separated and suspended in sterile water. This colony was found to have G418 resistance as shown in Table 2 (fused cell markers). Thus, the strain obtained as this colony is identified as a fused yeast formed from the above-mentioned two yeast strains. The reason for this conclusion is as follows.

TMS-34, a parent strain of the fused yeast FKO-3, has the phenotype of leu⁻, HIS⁺, G418ʳ. TMM-23, the other parent strain, is an integrant derived from AH22 strain wherein the HSA gene has been incorporated in the LEU2 gene locus thereof, and its phenotype is LEU⁺, his⁻, G418ˢ. Consequently, the phenotype of the strain occurring due to fusion of the above-mentioned combination of the two parent strains is expected to be LEU⁺, HIS⁺, G418ʳ.

Immediately after being isolated as a single colony, the fused yeast of the present invention (FKO-3) was subjected to shaking culture at 30°C for 72 hours in a test tube containing YPD medium or YPG medium, followed by measurement of HSA production by the ELISA method. The results are summarized in Table 6. Table 6 also shows the results of measurement of HSA production by the ELISA method after shaking culture of the parent strains TMS-34 and TMS-23 under the same conditions as above. Use of galactose as a sugar source gave a higher production than with glucose. HSA production by the fused cell when using galactose as a sugar source was higher than that by the parent strains. Since the transcription unit of the HSA gene of TMM-23, a parent of FKO-3, has a GAL1 promoter, it is inferred that some part of the HSA gene is expressed constitutively and the rest is expressed inductively in FKO-3.

Also measured was HSA production by the fused yeast after 8 subcultures (once daily) on a plate of YPD medium, a nonselective medium (30°C). The results are shown in Table 7. As is evident from these results, HSA production showed substantially no reduction even after long-term subculture in the nonselective medium.

Also, 10 single clones were isolated from the clones formed after 8 subcultures (once daily) on a plate of YPD medium, a nonselective medium, and marker stability was determined. The results are shown in Table 8. As is evident from these results, the marker gene was stably retained even after long-term subculture in the nonselective medium.

Example 3

The properties of the parent strains used for cell fusion in Example 3 are as shown in Table 1.

Protoplasts of TMS-34 and THBS were fused to yield fused cells of TMS-34 and THBS (referred to as FKO-4) in the same manner as in Example 1. The total colony count was 49.

Fused cells were separated on a selection medium free of leucine and histidine. The single colony was separated and suspended in sterile water. This colony was found to have G418 resistance as shown in Table 2 (fused cell markers). Thus, the strain obtained as this colony is identified as a fused yeast formed from the above-mentioned two yeast strains. The reason for this conclusion is as follows:

TMS-34, a parent strain of the fused yeast FKO-4, is an integrant derived from AH22 strain wherein the HSA gene has been incorporated in the HIS4 and TRP1 gene loci thereof. Its phenotype is leu⁻, HIS⁺, G418$^r$. THBS, the other parent strain, is an integrant derived from AH22 strain wherein the HBsAg gene has been incorporated in the LEU2 gene locus thereof, and its phenotype is his⁻, LEU⁺, G418$^s$. Consequently, the phenotype of the strain occurring due to fusion of the above-mentioned combination of the two parent strains is expected to be LEU⁺, HIS⁺, G418$^r$.

Immediately after being isolated as a single colony, the fused yeast of the present invention (FKO-4) was subjected to shaking culture at 30°C for 72 hours in a test tube containing YPD medium, followed by measurement of HSA production by the ELISA method and of HBsAg production by the RPHA method. The results are summarized in Table 9. Table 9 also shows the results of measurement of HSA production by the ELISA method and of HBsAg production by the RPHA method after shaking culture of the parent strains TMS-34 and THBS under the same conditions as above. HSA production by the fused cells remained almost unchanged in comparison with the parent strains.

Also measured were HSA production by the ELISA method and of HBsAg production by the RPHA method after the fused yeast was subjected to 8 subcultures (once daily) on a plate of YPD medium, a nonselective medium. The results are shown in Table 10. As is evident from these results, HSA production showed substantially no reduction even after long-term subculture in the nonselective medium.

Also, 10 single clones were isolated from the clones formed after 8 subcultures (once daily) on a plate of YPD medium, a nonselective medium, and marker stability was determined. The results are shown in Table 11. As is evident from these results, the marker gene was stably retained even after long-term subculture in the nonselective medium.

In accordance with the present invention, foreign protein production efficiency can be improved noticeably since a fused yeast from two parent strains that express the same foreign protein permits increase in foreign protein production.

The present invention also makes it possible to produce two or more foreign proteins by a fused yeast produced from two parent strains that express different foreign proteins. This has a potential for considerable cost reduction, since if a strain producing a protein at a low cost and another strain producing another protein at a high cost are fused, it gives a strain capable of producing both proteins. In addition, appropriate combination of extracellular secretion and intracellular production will minimize mutual contamination of foreign proteins.

Table 1

| Properties of parent strain[1] used for cell fusion | | | | | | | | | | |
|---|---|---|---|---|---|---|---|---|---|---|
| Example | parent strain | plasmid | gene in plasmid | | | | | phenotype of parent strain | HSA production (mg/l) | HBsAg production ($2^n$) |
| | | | promoter | secretion signal | structural gene | terminator | target locus[2] | | | |
| 1 | TMS-34 | PMS008 PHO011 | GAP-DH GAP-DH | SUC2 SUC2 | HSA HSA | PHO5 PHO5 | HIS4 TRP1 | $HIS^+$, $leu^-$, $G418^r$ | 30.0 | - |
| | TMM-21 | PMM006 | GAP-DH | SUC2 | HSA | PHO5 | LEU2 | $his^-$, $LEU^+$, $G418^s$ | 22.5 | - |
| 2 | TMS-34 | PMS008 PHO011 | GAP-DH GAP-DH | SUC2 SUC2 | HSA HSA | PHO5 PHO5 | HIS4 TRP1 | $HIS^+$, $leu^-$, $G418^r$ | 30.0 | - |
| | TMM-23 | PMM010 | GAL1 | mHSA[3] | HSA | PHO5 | LEU2 | $his^-$, $LEU^+$, $G418^s$ | 24.8 | - |
| 3 | TMS-34 | PMS008 PHO011 | GAP-DH GAP-DH | SUC2 SUC2 | HSA HSA | PHO5 PHO5 | HIS4 TRP1 | $HIS^+$, $leu^-$, $G418^r$ | 22.5 | - |
| | THBS | pINT/HB | GAP-DH | none | HBsAg | PHO5 | LEU2 | $HIS^-$, $LEU^+$, $G418^s$ | - | 4 |
| Note: | | | | | | | | | | |

1) strain wherein a heterologous gene (in this case HSA or HBsAg) is incorporated in host chromosome
2) the region corresponding to chromosome of the host ( = foreign protein incorporation site)
3) synthetic signal modified from HSA signal (disclosed in Japanese Patent Application No. 103339.1988)

EP 0 409 156 A1

Table 2

|  | Fused yeast | LEU2 | HIS4 | G418$^r$ |
|---|---|---|---|---|
| Example 1 | FKO-1 (TMS-34 x TMM-21) | + | + | + |
| Example 2 | FKO-3 (TMS-34 x TMM-23) | + | + | + |
| Example 3 | FKO-4 (TMS-34 x THBS) | + | + | - |

Table 3

| HSA Production of fused yeast FKO-1 and its parent strains cultured immediately after isolation as a single colony | |
| --- | --- |
| Clone No. | HSA production (mg/l) |
| FKO-1-01 | 51.0 |
| FKO-1-02 | 49.5 |
| FKO-1-03 | 38.5 |
| FKO-1-07 | 36.0 |
| FKO-1-10 | 39.0 |
| FKO-1-11 | 39.0 |
| FKO-1-12 | 37.0 |
| FKO-1-13 | 37.5 |
| FKO-1-16 | 45.0 |
| FKO-1-20 | 41.3 |
| TMS-34 | 30.0 |
| TMM-21 | 22.5 |

Table 4

HSA production of fused yeast FKO-1 after long-term
subculture in      nonselective medium

| Clone No. | HSA production after long-term subculture (mg/l) | HSA production before long-term subculture (mg/l) | |
|-----------|---------------------------------|----------------------------------|---|
| FKO-1-01 | 40.2 | 51.0 | |
| FKO-1-02 | 50.5 | 49.5 | |
| FKO-1-07 | 34.5 | 36.0 | |
| FKO-1-11 | 39.0 | 39.0 | same results as in Table 3 |
| FKO-1-16 | 52.5 | 45.0 | |
| TMS-34 | 33.0 | 30.0 | |
| TMM-21 | 23.8 | 22.5 | |

Table 5

| Marker stability of FKO-1 in nonselective medium | | | |
|------------------------------------------------|------|-------------|--------------|
| Clone | Colony formation frequency | | |
| | YPD | $YPDGen_{100}$ | YNB(w/o) + D |
| FKO-1-1 | 10/10 | 10/10 | 10/10 |
| FKO-1-3 | 10/10 | 9/10 | 10/10 |
| FKO-1-11 | 10/10 | 10/10 | 10/10 |
| In Table, D means glucose. YNB(w/o) : not including Trp, His and Leu $YPDGen_{100}$ : YPD added with 100 µg/ml of G418 | | | |

Table 6

| HSA Production of fused yeast FKO-3 and its parent strains cultured immediately after isolation as a single colony | | |
|---|---|---|
| Clone No. | sugar | HSA production (mg/l) |
| FKO-3-01 | dextrose | 26.6 |
| FKO-3-01 | galactose | 47.3 |
| FKO-3-09 | dextrose | 27.8 |
| FKO-3-09 | galactose | 41.1 |
| FKO-3-11 | dextrose | 26.3 |
| FKO-3-11 | galactose | 44.5 |
| FKO-3-12 | dextrose | 25.0 |
| FKO-3-12 | galactose | 45.0 |
| FKO-3-15 | dextrose | 28.6 |
| FKO-3-15 | galactose | 43.8 |
| FKO-3-16 | dextrose | 26.2 |
| FKO-3-16 | galactose | 44.7 |
| TMS-34 | dextrose | 31.0 |
| TMS-34 | galactose | 30.0 |
| TMM-23 | dextrose | below detection limit |
| TMM-23 | galactose | 24.8 |

Table 7

| HSA production of fused yeast FKO-3 after long-term subculture in nonselective medium | | | |
|---|---|---|---|
| Clone No. | sugar | HSA production after long-term subculture (mg/l) | HSA production before long-term subculture (mg/l) |
| FKO-3-01 | dextrose | 22.9 | 26.6 |
| FKO-3-01 | galactose | 42.1 | 47.3 |
| FKO-3-09 | dextrose | 28.8 | 27.8 |
| FKO-3-09 | galactose | 51.8 | 41.1 |
| FKO-3-11 | dextrose | 22.1 | 26.3 |
| FKO-3-11 | galactose | 40.0 | 44.5 |
| FKO-3-15 | dextrose | 25.0 | 28.6 |
| FKO-3-15 | galactose | 40.9 | 43.8 |
| TMS-34 | dextrose | 39.3 | 31.0 |
| TMS-34 | galactose | 29.0 | 30.0 |
| TMM-23 | dextrose | below detection limit | below detection limit |
| TMM-23 | galactose | 18.0 | 24.8 |

Table 8

| Marker stability of FKO-3 in nonselective medium | | | |
|---|---|---|---|
| Clone | Colony formation frequency | | |
| | YPD | YPDGen$_{100}$ | YNB(w/o) + D |
| FKO-3-1 | 10/10 | 10/10 | 10/10 |
| FKO-3-9 | 10/10 | 10/10 | 10/10 |
| FKO-3-11 | 10/10 | 10/10 | 10/10 |
| In Table, D means glucose. YNB(w/o) : not including Trp, His and Leu YPDGen$_{100}$ : YPD added with 100 $\mu$g/ml of G418 | | | |

Table 9

| HSA Production of fused yeast FKO-4 and its parent strains cultured immediately after isolation as a single colony | | |
|---|---|---|
| Clone No. | HSA production (mg/l) | HBsAg production ($2^n$) |
| FKO-4-01 | 24.8 | 3 |
| FKO-4-04 | 19.5 | 3 |
| FKO-4-07 | 17.9 | 3 |
| FKO-4-11 | 20.0 | 4 |
| FKO-4-12 | 21.2 | 3 |
| FKO-4-13 | 22.5 | 4 |
| FKO-4-16 | 23.5 | 3 |
| FKO-4-18 | 18.5 | 2 |
| FKO-4-19 | 22.0 | 4 |
| FKO-4-20 | 23.0 | 4 |
| TMS-34 | 22.5 | below detection limit |
| THBS | below detection limit | 4 |
| 72 hr shaking culture at 30° C in a YPD-containing test tube (3 ml) HSA production was measured by the ELISA method. HBsAg production was measured by the RPHA method. | | |

Table 10

| HSA production of fused yeast FKO-4 after long-term subculture in the nonselective medium | | | | |
|---|---|---|---|---|
| Clone No. | after long-term subculture | | before long-term subculture | |
| | HSA production (mg/l) | HBsAg production ($2^n$) | HSA production (mg/l) | HBsAg production ($2^n$) |
| FKO-4-01 | 22.6 | 4 | 24.8 | 3 |
| FKO-4-04 | 20.5 | 3 | 19.5 | 3 |
| FKO-4-07 | 20.0 | 3 | 17.9 | 3 |
| FKO-4-13 | 19.5 | 4 | 22.5 | 4 |
| FKO-4-19 | 19.0 | 3 | 22.0 | 4 |
| TMS-34 | 23.8 | below detection limit | 22.5 | below detection limit |
| THBS | below detection limit | 4 | below detection limit | 4 |

Table 11

| Marker stability of FKO-4 in nonselective medium | | | |
|---|---|---|---|
| Clone | Colony formation frequency | | |
| | YPD | YPDGen$_{100}$ | YNB(w/o) + D |
| FKO-4-04 | 10/10/10 | 10/10/10 | 10/10/10 |
| FKO-4-13 | 10/10/10 | 10/10/10 | 10/10/10 |
| FKO-4-19 | 10/10/10 | 10/10/10 | 10/10/10 |
| In Table, D means glucose. YNB(w/o) : not including Trp, His and Leu YPDGen$_{100}$ : YPD added with 100 $\mu$g/ml of G418 | | | |

## Claims

1. A fused yeast possessing the capabilities of producing a foreign protein of both parent yeasts, which is produced by fusing a first yeast capable of producing a foreign protein wherein a DNA sequence encoding the foreign protein has been incorporated in the chromosome thereof, and a second yeast capable of producing a foreign protein wherein a DNA sequence encoding the foreign protein has been incorporated in the chromosome thereof.

2. A fused yeast as claimed in Claim 1 wherein the parent yeasts are derived from Saccharomyces cerevisiae .

3. A fused yeast as claimed in Claim 1 wherein a protein encoded by a DNA sequence incorporated in a first yeast and a protein encoded by a DNA sequence incorporated in a second yeast are the same.

4. A fused yeast as claimed in Claim 3 wherein a protein encoded by a DNA sequence incorporated in a first and a second yeasts is serum albumin (HSA).

5. A fused yeast as claimed in Claim 3 which is produced by fusing a first yeast obtained by transformation of a host yeast cell with a plasmid containing leucine synthetic pathway gene LEU2 in a sequence homologous to the chromosome sequence of the host yeast cell and an HSA structural gene as a foreign protein structural gene; and a second yeast obtained by transformation of a host yeast cell with a plasmid containing histidine synthetic pathway gene HIS4 in a sequence homologous to the chromosome sequence of the host yeast cell and an HSA structural gene as a foreign protein structural gene, and a plasmid containing tryptophan synthetic pathway gene TRP1 in a sequence homologous to the chromosome sequence of the host yeast cell, an HSA structural gene as a foreign protein structural gene and a G418-resistant gene as a yeast selection marker gene.

6. A fused yeast as claimed in Claim 3 which is leucine/histidine-nonrequiring and G418-resistant.

7. A fused yeast as claimed in Claim 1 wherein a protein encoded by a DNA sequence incorporated in a first yeast and a protein encoded by a DNA sequence incorporated in a second yeast are different.

8. A fused yeast as claimed in Claim 7 wherein a protein encoded by a DNA sequence incorporated in a first yeast is serum albumin (HSA) and a protein encoded by a DNA sequence incorporated in a second yeast is HBsAg.

9. A fused yeast as claimed in Claim 7 which is produced by fusing a first yeast obtained by transformation of a host yeast cell with a plasmid containing leucine synthetic pathway gene LEU2 in a sequence homologous to the chromosome sequence of the host yeast cell and an HBsAg structural gene as a foreign protein structural gene; and a second yeast obtained by transformation of a host yeast cell with a plasmid containing histidine synthetic pathway gene HIS4 in a sequence homologous to the chromosome sequence of the host yeast cell and an HSA structural gene as a foreign protein structural gene, and a plasmid containing tryptophan synthetic pathway gene TRP1 in a sequence homologous to the chromosome sequence of the host yeast cell, an HSA structural gene as a foreign protein structural gene and a G418-resistant gene as a yeast selection marker gene.

10. A fused yeast as claimed in Claim 7 which is leucine/histidine-nonrequiring and G418-resistant.

11. A method of producing a fused yeast capable of producing a foreign protein of both parent yeasts, characterized by fusion between a first yeast capable of producing a foreign protein wherein a DNA sequence encoding the foreign protein has been incorporated in the chromosome thereof and a second yeast capable of producing a foreign protein wherein a DNA sequence encoding the foreign protein has been incorporated in the chromosome thereof.

12. A method of producing a fused yeast as claimed in Claim 11 wherein a protein encoded by a DNA sequence incorporated in a first yeast and a protein encoded by a DNA sequence incorporated in a second yeast are the same.

13. A method of producing a fused yeast as claimed in Claim 11 wherein a protein encoded by a DNA sequence incorporated in a first yeast and a protein encoded by a DNA sequence incorporated in a second yeast are different.

14. A method of producing a foreign protein, characterized in that the fused yeast described in (1) above is cultivated to produce a foreign protein, which is then harvested.

_Fig - 1_

(a)

pMM006
8.1kb

(b)

pHO011
8.4kb

(c)

pMS008
11.9kb

Fig - 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DERWENT/WPIL, accession no. 86-128921[20], Derwent Publications Ltd, London, GB; & JP-A-61 067 478 (SUNTORY LTD) | 1-14 | C 12 N 15/04 |
| A | BIOTECHNOLOGY AND GENETIC ENGINEERING REVIEWS, vol. 3, September 1985, pages 377-416, Intercept Ltd, Newcastle upon Tyne, GB; S.M. KINGSMAN et al.: "Heterologous gene expression in Saccharomyces cerevisiae" | | |
| A | BIOTECHNOLOGY AND GENETIC ENGINEERING REVIEWS, vol. 4, September 1986, pages 1-38, Intercept Ltd, Newcastle upon Tyne, GB; S.L. STURLEY et al.: "Genetic manipulation of commercial yeast strains" | | |

**TECHNICAL FIELDS SEARCHED (Int. Cl.5)**

C 12 N

The present search report has been drawn up for all claims

| Place of search | Date of completion of the search | Examiner |
|---|---|---|
| THE HAGUE | 05-10-1990 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)